# EUROPÄISCHE PATENTANMELDUNG

(11) **EP 1 978 358 A2**
(43) Veröffentlichungstag der Anmeldung: **08.10.2008**
(21) Anmeldenummer: 08103320.1
(22) Anmeldetag: 02.04.2008
(51) Int. Cl.: G01N 33/00, C12M 1/34, C12Q 1/02, G01N 27/414

(54) **Sensor zur Erfassung eines toxischen oder gefährlichen Gasgemisches und Betriebsverfahren**

(30) Priorität: 05.04.2007 DE 102007016629
(71) Anmelder: SIEMENS AKTIENGESELLSCHAFT, 80333 München (DE)
(72) Erfinder: Lehmann, Mirko, 9642 Ebnat-Kappel (CH); Fleischer, Maximilian, 85635, Höhenkirchen (DE); Simon, Elfriede, 80639, München (DE)

(57) **Zusammenfassung**

Sensor zur Erfassung eines Gasgemisches, welches im Wesentlichen aus Luft besteht und ein oder mehrere Gase enthält, die auf Lebewesen eine nachteilige Wirkung zeigen, bestehend aus: einem Sensorchip aus Silizium zur Auslesung von mindestens einem Signal, das an einer sensitiven Substanz bei vorliegendem einen oder mehreren Zielgasen im Messgas generiert wird, einer auf dem Sensorchip aufgebrachten sensitiven Substanz, die aus lebenden Zellen besteht, welche auf Zielgase ansprechen, einer Signalverarbeitungseinheit zur Auswertung der Signale des Si-Chips.

## Beschreibung

Die Erfindung betrifft einen Sensor für die Detektion von in Gasgemischen enthaltenen toxischen oder gefährlichen Gasen, sowie ein Betriebsverfahren.

Die Erfassung soll eine toxische oder anderweitig als gefährlich einzustufende Belastung ermitteln ohne dass in einem Gasgemisch eine Differenzierung einzelner Gase vorzunehmen ist. Die in der Umwelt vorhandene Gasatmosphäre ist in der Regel ein Gasgemisch mit mehreren unterschiedlichen Gasen. Zum Nachweis einer Zusammensetzung eines Gasgemisches sind bisher entsprechende auf jeweils ein Gas ausgerichtete Gassensoren notwendig. Diese messen die Anwesenheit eines Zielgases und evtl. auch dessen Konzentration.

Der bisherige Ansatz, Gase festzustellen und zu kategorisieren, kann durchaus an seine Grenzen stoßen, wenn unterschiedlichste Varianten von toxischen oder gefährlichen Gasen detektiert werden müssen und somit ein Messsystem sehr komplex gestaltet sein muss.

Bisher war für die Überwachung auf gefährliche Substanzen die genaue Kenntnis der Zielsubstanz/en notwendig. Damit können Gassensoren oder eine Mehrzahl von Gassensoren eingesetzt werden, um diese Subtanzen einzeln zu detektieren.

Es gab schon erste Ansätze, Lebewesen als Indikator für die Präsenz gefährlicher Substanzen in Luft einzusetzen. Ein klassisches Beispiel sind die seit Langem im Untertagebau eingesetzten Kanarienvögel, die eine große Empfindlichkeit auf giftige Gase besitzen und dadurch Menschen schützten können.

Ansätze, Lebewesen und ihre biologischen Rezeptoren als Sensoren einzusetzen sind bekannt. So können beispielsweise die Antennen von Kartoffelkäfern in elektrischen Kontakt mit dem Gate von Feldeffekt-Transistoren gebracht werden. Die Reaktion einer hochempfindlichen Käferantenne auf Gase oder Gasgemische kann über den Source-Drain-Strom von FETs ausgelesen werden.

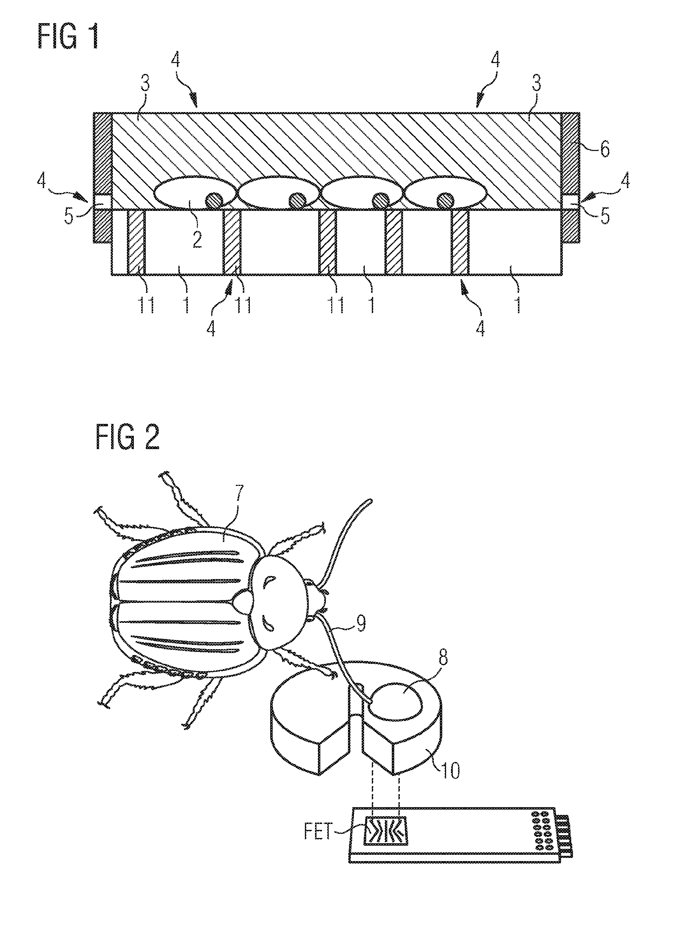

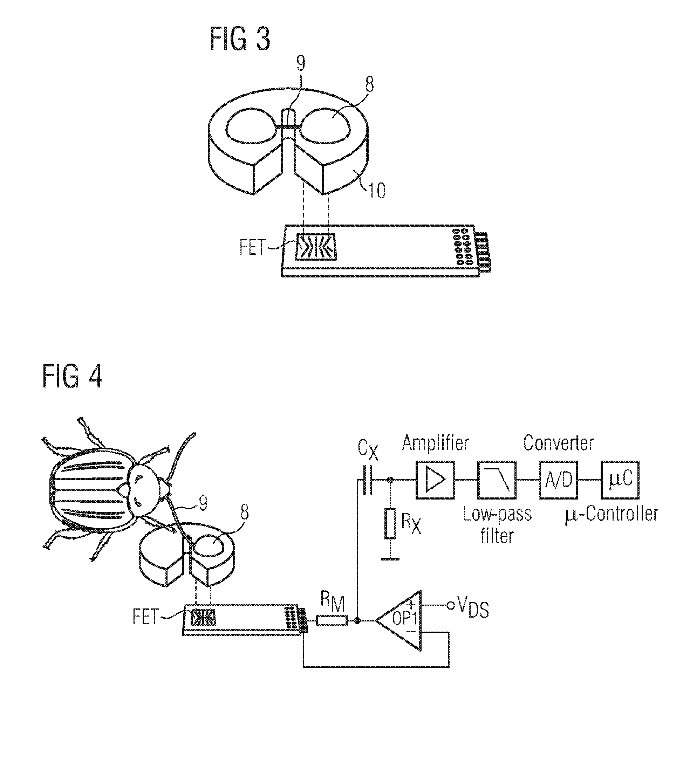

Die Figuren 2 und 3 zeigen jeweils ein Schema einer Versuchsanordnung mit einer Halterung 10 für den Fühler 9 von Kartoffelkäfern 7. Dabei ist nach Figur 2 der gesamte Kartoffelkäfer bei der Detektion vorhanden, während nach Figur 3 ein separater Fühler 9 des Kartoffelkäfers 7 eingesetzt wird. Die Messung wird mit Hilfe eines Elektrolyten 8 ausgeführt. Eine Auslesung von generierten Messsignalen geschieht über mindestens einen FET, der entsprechend an einer Halterung befestigt ist.

Figur 4 zeigt eine Anordnung entsprechend Figur 2 mit der beispielhaften Darstellung einer elektronischen Auswerteschaltung mit verschiedenen Verstärker-, Filter- und Wandlereinheiten.

Weiterhin sind Ansätze, Zellen an sich als biologische Rezeptoren einzusetzen, im medizinischen Bereich z.B. in der Onkologie zur Verbesserung der Behandlung von Patienten mit Krebserkrankungen bekannt.

In der Veröffentlichung: Lehmann, M., Baumann, W., Brischwein, M., Gahle, H.J., Freund, I., Ehret, R., Drechsler, S., Palzer, H., Kleintges, M., Sieben, U., Wolf, B., 2001, "Simultaneous measurement of cellular respiration and acidification with a single CMOS ISFET, Biosensors & Bioelectronics", 16, Seiten 195-203, werden im Wesentlichen der pH-Wert und der Sauerstoffpartialdruck als Parameter für den Fortbestand von lebendem Gewebe beobachtet.

Weiterhin werden in der DE 19827957 C2 ein Verfahren und eine Vorrichtung zur Messung einer Zustandsgröße beschrieben.

Die DE 19920811A1 offenbart eine Vorrichtung zur Durchführung von Untersuchungen an Zellkulturen.

Und die DE 10028692 A1 offenbart ein Verfahren zur Untersuchung von mit Membran umschlossenen Biokompartimenten.

In den hier aufgeführten Patentanmeldungen bzw. Veröffentlichungen sind Sensorchips und Systeme beschrieben, die generell entweder auf die Antwort von Zellen auf bekannte Flüssigkeiten, wie z.B. bei der Wirkstoffsuche in der Medikamentenentwicklung, oder auf die Antwort von Zellen auf unbekannte Flüssigkeiten, wie z.B. bei der Überwachung der Wasserqualität, abzielen.

Der Erfindung liegt die Aufgabe zugrunde, einen Sensor und ein Betriebsverfahren zu beschreiben, womit die Erfassung von Signalen lebender Zellen als Rezeptoren bei in einem Messgas vorhandenen toxischen oder gefährlichen Gasen oder Gasgemischen möglich ist.

Die Lösung dieser Aufgabe geschieht durch die jeweilige Merkmalskombination der Ansprüche 1 bzw. 23.

Vorteilhafte Ausgestaltungen sind den Unteransprüchen zu entnehmen.

Der Erfindung liegt die Erkenntnis zugrunde, dass mit diesem Ansatz im Gegensatz zu bisherigen Ansätzen der Gassensorik Aufgabenstellungen gelöst werden, die eine selektive Detektion eines Einzelgases nicht notwendig machen, sondern eine Untersuchung einer Umgebung mit einem Gasgemisch ermöglichen, mit einer Aussage über die Schädlichkeit für Lebewesen. Dabei wird die Erfassung nicht auf eines oder mehrere Gase eingeengt sondern ein beliebig großes Spektrum möglicher Gase betrachtet.

Anwendungen liegen z.B. in der Umweltmesstechnik. Dort besteht oft die Problematik darin die Belastung einer Umgebungsatmosphäre zu messen und bewerten zu können. Es kann festgestellt werden, ob in der Atmosphäre z.B. eine Belastung durch Verkehr, Belastung durch industrielle Abgase oder auch Industrielle Abluft vorhanden sind oder allgemein Bestandteile vorhanden sind, die schädlich für Leben sind, wobei das Hauptschwergewicht hier auf eine Schädlichkeit für Menschen gelegt wird. Andere Anwendungen liegen in der Terrorismusabwehr oder der Arbeitsplatzsicherheit mit der Überwachung gefährlicher Chemikalien bzw. Gemische, wobei damit korrelierenden Gase oder Gasgemische zu detektieren sind. Dabei wird erfasst, ob sich für Leben schädliche Substanzen in der Umgebung befinden, ohne dass die Zielsubstanzen explizit bekannt sein müssen.

Vorteilhafte Ausbildungen sind insbesondere wie folgt:

Die Zellen befinden sich direkt auf / in unmittelbarer Nähe des Chipbereiches, der die Zellsignale ausliest.

Die Signale der lebenden Zellen werden von Elektroden aufgenommen, die diese zu von den Zellen beabstandeten Chipbereichen zur Signalerfassung weiterleiten, d. h. die Zellsignale auslesen.

Es ist vorteilhaft eine feinporige Membran mit Porenweiten von typischerweise 0,1-10 µm zu verwendet, die den Zutritt von Gasen zum Sensor zulässt, Staub, Aerosole und Fremdbakterien sowie Viren aber fernhält. Diese Membran kann zudem so dimensioniert werden, dass ein ausreichender Gaszutritt zu den Zellen gewährleistet wird, durch die teilweise Bedeckung der Oberfläche jedoch ein Feuchtigkeitsverlust reduziert wird.

In dem Gehäuse befinden sich Vorrichtungen, die ein Überleben der Zellen über längere Zeiten ermöglichen. Diese Vorrichtungen sorgen z.B. für eine Versorgung der Zellen mit ausreichend Feuchtigkeit, um ein Austrocknen zu Vermeiden und stellen eine Nährstoffversorgung zum Überleben der Zellen sicher. Wenn Zellen verwendet werden, die Licht zum Überleben benötigen, was z.B. auf Photosynthese basierende Organismen sind, dann ist auch eine Versorgung mit Licht des benötigten Spektralbereichs sicherzustellen.

Der Sensorchip ist dergestalt aufgebaut, dass die Zellen auf dem Chip in eine gasdurchlässige Matrix eingebettet sind, die eine bessere Verankerung der Zellen bewirkt und die Zellen auf der Sensoroberfläche fixiert.

Vorteilhaft ist es, die Matrix zur Sicherstellung von ausreichender Feuchte auszustatten.

Eine weitere vorteilhafte Ausgestaltung sieht vor, dass die Matrix die Sicherstellung der Nährstoffversorgung der Zellen bewirkt.

Eine Ausgestaltung betrifft die Matrix, die einen Wachstumshemmer (Zytostatika) enthält, um eine Wucherung der Zellen zu verhindern.

Es ist weiterhin vorteilhaft die Matrix mit Bakteriziden, z.B. Antibiotika und Fungiziden z.B. Antimykotika zu versetzen, um die Ansiedelung von Fremdbakterien und Pilzen und Sporen zu verhindern.

Besondere Ausgestaltung, dass die Matrix ein Natriumazid, Streptomycin, Penicillin enthält um den Befall von Fremdbakterien zu verhindern.

Besondere Ausgestaltung, dass die Matrix die für das überleben der Zellen notwenigen Substanzen enthält.

Es ist besonders vorteilhaft, dass die Matrix Substanzen wie z.B. Proteine und Enzyme enthält, z. B. durch Zusatz von fetalem Serum, normalen Serum oder Glutamin.

Weiterhin kann auf der Matrix direkt eine feinporige Membran mit Porenweiten typischerweise zwischen 0,1 und 10 µm angebracht sein, die den Zutritt von Gasen zum Sensor zulässt, Staub, Aerosole und Fremdbakterien sowie Viren aber fernhält.

Da die lebenden Zellen nur in einer flüssigen Umgebung überleben können, muss das Gas vom gasförmigen Zustand in den flüssigen Zustand übergehen, um zu den Zellen zu gelangen. Die Löslichkeit von Gas zu Flüssigkeit wird über das Henrysche Gesetz bestimmt. Hier ist zu beachten, dass die Diffusion in Gasen sehr viel schneller abläuft als in Flüssigkeiten. Die Diffusionszeit von H+ Protonen für 1mm in Flüssigkeit beträgt etwa 10s. Je größer die Gasmoleküle desto länger wird diese Zeit. Für eine schnelle Ansprechzeit des Sensors auf z.B. Giftgas etc ist deshalb der Weg von der Grenze Gas/Flüssigkeit zu den Zellen auf dem Sensor so kurz wie möglich zu gestalten. Des Weiteren muss dafür gesorgt werden, dass ein solcher Zugang vorhanden ist, da bei den normalen Zell Sensor Systemen ein solcher Zugang eher ausgeschlossen werden soll, um die Zellen und die Flüssigkeit nicht zu kontaminieren.

Es ist vorteilhaft, dass eine Entfernung zwischen den lebenden Zellen und der Gasumgebung im betrachteten System, also der Weg von der Grenze Gas/Flüssigkeit zu den lebenden Zellen unter 1mm liegt, vorzugsweise unter 500 µm.

Eine Möglichkeit der Messgaszufuhr zum Rezeptor bzw. zur gassensitiven Einheit, hier die lebenden Zellen, besteht in der Freiätzung des Silizium-Chips an der Rückseite in kleinen Bereichen mit einem Durchmesser vom 0,1 mm Bereich bis hinunter zu etwa 1-10 µm, so dass das Gas die Zelle schnell erreicht und trotzdem die Höhe der Flüssigkeit über den Zellen einigermaßen frei gewählt werden kann. Wenn die Löcher z.B. in klassischer nasschemischer Ätztechnik im Silizium erzeugt werden, ist zu beachten, dass diese einen V-förmigen Querschnitt aufweisen. Mit der Angabe des Durchmessers ist dann die engste Stelle des Loches gemeint. Die Wahl der Größe und Anzahl der "Löcher" müsste in Abhängigkeit der Höhe des Druckes der Flüssigkeit geschehen, die sich im Wesentlichen aus der Höhe und dem Durchfluss ergibt und so gewählt werden kann, dass die Flüssigkeit nicht aus den Löchern heraustritt. Des Weiteren ist es sinnvoll die Löcher so zu gestalten, dass die Zellen nicht durch die Löcher durchtreten, dies kann z.B. auch mit einer Membran auf der Si-Oberfläche bewerkstelligt werden. Eine weitere ähnliche Möglichkeit sieht die Einbringung von Durchgängen in die Wände von Packages vor.

Auch bei dieser Ausführung, bei der das Messgas "von unten her" rasch zu den Zellen gelangt, ohne Zeit aufwändige Diffusionswege durch die Flüssigkeit zurücklegen zu müssen, kann es sinnvoll sein, eine der oben genannten feinporigen Membranen zu verwenden, um Fremdpartikel fernzuhalten. Diese Membran ist dann über den Poren angebracht.

Alternativ bedient man sich einer fluidischen Lösung, in dem die Wechselwirkung Gas und Flüssigkeit nicht unmittelbar über dem Sensorchip passiert, sondern in einem Fluidkanal vor dem Sensorchip, der sehr dünn ausgestaltet ist (10 µm bis 1 mm Durchmesser). Dieser Fluidkanal ist sehr lang, damit viel Flüssigkeit mit dem Gas in Kontakt kommt. Das Trennmaterial des Fluidkanals ist dann natürlich gasdurchlässig, damit das Gas in die Flüssigkeit gelangen kann. Diese mit dem Gas versehene Flüssigkeit wird dann an die Zellen gebracht und damit das Gas den Zellen zugeführt. Zur Bewegung der Flüssigkeit kann eine an den Fluidkanal angeschlossene Pumpe verwendet werden, um die mit dem Gas "kontaminierte" Flüssigkeit über den Sensorchip zu führen. Alternativ kann die Flüssigkeit auch über andere Varianten wie über eine hydrostatische Lösung, über die Schwerkraft bewegt werden.

Wenn eine Lösung verwendet wird, bei der eine aktive Spülung der Zellen mit einem Fluid geschieht, kann es vorteilhaft sein, das Spülen der Zellen zwischenzeitlich zu unterbrechen. Während der Zeit, in der Zellen mit der mit dem Gas beaufschlagten Flüssigkeit gespült werden, findet der Hintransport des Gases zu den Zellen statt. Gleichzeitig werden aber auch Stoffwechselprodukte der Zellen, die mit dem Si-Chip ausgelesen werden abtransportiert und können daher nur mit reduzierter Empfindlichkeit gemessen werden. Wenn nun der Fluss gestoppt wird, dann reichern sich diese Stoffwechselprodukte an. Es ist nun möglich Änderungen an den Zellen über die Messung dieser Stoffwechselprodukte empfindlicher zu detektieren. Zum anderen kann dies auch zu einer Stabilisierung des Messverfahrens genutzt werden, wenn nicht mehr der Absolutgehalt der Stoffwechselprodukte der Zellen gemessen wird sondern nur der relative Anstieg von deren Konzentration. Bei dieser Variante wird von den Aufnehmern im Silizium nicht mehr gefordert, dass diese eine stabile Basislinie aufweisen. Es reicht aus, wenn von den Aufnehmern im Silizium eine Änderung der Konzentration an Stoffwechselprodukten gemessen wird.

Da jede Zelle unterschiedlich auf die äußeren Gase anspricht, ist es vermutlich sinnvoll nicht nur einen Sensorchips mit einem einzelnen Zelltypus sondern mehrere zu verwenden, wobei die Sensorchips sich durch die andere Zellen unterscheiden. Wenn man z.B. an ein Gerät zur Überwachung von Orten denkt, so ist es ziemlich unwahrscheinlich, dass es eine Zelle gibt, die auf alle Giftgase in geeigneter Weise anspricht. D.h. man hat ein System mit mindestens zwei Sensorchips mit unterschiedlichen Zelltypen, deren Signale miteinander verarbeitet einen Alarm auslösen können. Alternativ können aus Vereinfachungsgründen des Aufbaus auch verschiedene Zelllinien auf einem Chip vorgesehen werden, deren Summensignal dann ausgelesen wird.

Eine Zelllinie, die in einer bestimmten Dichte auf dem Auslesechip aufgebracht ist, wird eine spezifische Schadstoffkonzentration aufweisen, bei der sie die stärksten Reaktionen zeigt. Um den Messbereich eines Zellsensors zu erweitern, wird es daher sinnvoll sein, verschiedene Einzelsensoren vorzusehen, die mit unterschiedlichen Zelldichten versehen sind. Jeder Einzelsensor mit einer bestimmten Zelldichte ist so für einen bestimmten Konzentrationsbereich von Schadstoffen optimal sensitiv.

Das Sensorsystem ist mit einer Autokalibriereinheit ausgestattet um den Nullpunkt in regelmäßigen Zeitabständen auf die Kontrollsensoren einzustellen.

Um den Kontakt des zellbasierten Sensors mit gefährlichen und toxischen Gasen zu detektieren werden die Änderung einer oder mehrerer der für Zellen typischen Parameter wie Adhäsion, detektiert über eine Impedanzmessung, wie O₂-Verbrauch, detektiert über eine O₂ Messung, Ansäuerung, detektiert über eine pH-Wert Messung, Elektrische Aktivität, detektiert über eine Potentialänderung oder Bioluminiszenz, detektiert über eine Messung des ausgesendeten Lichtes, aufgenommen. Diese können dann mit den Signalen eines zweiten unbelasteten Zellsensors verglichen werden.

Bestimmung der des Zeitverlaufes der Änderung der Parameter und Auswertung der Parameterkinetik.

Bisherige Ansätze für Gebiete wie z.B. Umweltmesstechnik - Luft, Wasser - oder Terrorismusabwehr, bezogen auf Giftgas, basieren darauf, dass immer Sensoren für Einzelsubstanzen verwendet werden. Wenn viele Einzelsubstanzen in Frage kommen, müssen sehr viele Sensorelemente verwendet werden, die dann den Aufbau teuer und störanfällig machen.

Bei manchen Anwendungen sind die möglichen giftigen/störenden Stoffe entsprechender Substanzen nicht bekannt oder zu vielfältig, so dass der Ansatz über die Detektion von Einzelsubstanzen nicht gangbar ist.

Beides zusammen löst der beschriebene Ansatz. Durch die Verwendung von lebenden Zellen tritt ein Querschnittsrezeptor in Aktion, der eine Vielzahl von Substanzen bzw. deren Auswirkung auf lebende Zellen mit einem Sensorelement misst.

Zugleich besteht die Möglichkeit, durch geeignete Wahl der Zelllinie, die relative Verteilung der Empfindlichkeiten auf die unterschiedlichen schädigenden Stoffe der menschlichen Empfindlichkeit anzupassen.

Zellen benötigen die Einhaltung eines gewissen Temperaturbereiches, um leben zu können. Um den Einsatz eines erfindungsgemäßen Aufbaus in vielfältigen Umgebungen durchführen zu können, wird daher auch optional die Verwendung einer Heizung oder Kühlung zum Einhalten einer konstanten vorgegebenen Temperatur des Aufbaus vorgesehen.

Zellen weisen oft eine Begrenzte Lebensdauer auf. Wenn diese zu kurz für die Applikation ist, können mehrere gleichartige Chips vorgesehen werden. Dabei wird beispielsweise ein Chip zur Messung benutzt, während die anderen Chips nicht benutzt werden. Die Nicht-Benutzung der Chips wird dabei sichergestellt, indem z.B. keine Beaufschlagung der Chips mit dem Messgas geschieht, oder indem die Chips durch Verwendung eines Wachstumshemmers für die Zellen oder für die Wahl der lokalen Temperatur deaktiviert werden. Bei Hinweisen auf die Erschöpfung der Messfähigkeit des Chips oder nach einer fest vorgegebenen Zeit wird dann der nächste Chip benutzt. Hierzu werden die deaktivierenden Einflüsse entfernt und das Signal des derart aktivieren Chips ausgelesen.

An die Chips ist eine Auswerteeinheit angeschlossen. Diese wird eine Auswertung der Signale der einzelnen Chips vornehmen.

Wenn nur ein einzelner Chip ausgelesen wird, kann keine Aussage über die Art des die Zellen beeinflussenden Gases/der Gasgruppe getroffen werden, da nicht erkennbar ist, ob eine geringe Konzentration eines die Zellen stark beeinflussenden Gases oder eine höherer Konzentration eines die Zellen weniger stark beeinflussenden Gases vorliegt. Wenn die zwei die Zellen gleichermaßen stark beeinflussenden Gase vorliegen, kann keine Aussage getroffen werden, um welches Gas es sich handelt. Wenn nun mehrere verschiedene Chips vorhanden sind und betrieben werden, deren Zellen auf verschiedene Gase unterschiedlich reagieren, werden unterschiedliche Signale erhalten. Durch eine Auswertung der unterschiedlichen Signale in der Auswerteeinheit z.B. durch eine Regelbasierte Auswertung oder mit den Methoden der Mustererkennung, kann nun bei Kenntnis der Empfindlichkeiten der einzelnen Chips eine Klassifikation des Ereignisse in Hinblick auf eine Aussage über die Art der vorliegenden Gase/Gasgruppen getroffen werden.

Im Folgenden wird die Erfindung anhand von schematischen die Erfindung nicht einschränkenden Ausführungsbeispielen beschrieben:
- Figur 1: zeigt eine Anordnung entsprechend der Erfindung, wobei ein Sensor im Schnitt dargestellt ist,
- Figuren 2, 3 und 4: zeigen Anordnungen, die den Stand der Technik wiedergeben.

Das Sensorsystem wird zusammen mit einer Autokalibriereinheit betrieben, um den Nullpunkt in regelmäßigen Zeitabständen auf die Kontrollsensoren einzustellen. Der Sensor besteht aus mindesten drei mit Umgebungsluft/Messgas beaufschlagten separaten aber baugleichen Sensoreinheiten und drei mit unbelasteter Luft kontaktieren separaten aber baugleichen Einheiten. Eine aufgrund einer Störung, wie z.B. Kontamination mit Fremdzellen, ausfallende Einheit kann dann aus Plausibilitätsgründen (2 gleiche und 1 verschiedenes Reaktionsmuster) erkannt werden.

Das Sensorsystem wird in regelmäßigen Abständen mit neuen Zellsensoren bestückt, wie typischerweise alle 5-40 Tage.

Das Sensorsystem ist so aufgebaut, dass der Sensorchip einfach mit Hilfe eines seitlich angebrachten Stecksystems ausgetauscht werden kann.

Figur 1 zeigt einen Sensor in einer möglichen Querschnittsform. Mittig angeordnet sind sinnbildlich vier übergroße lebende Zellen in ovaler Form mit ebenso vergrößert dargestelltem kreisrunden jeweiligen Zellkern.

Die lebenden Zellen 2 liegen auf der oberen Seite des Silizium-Chips 1 auf, wobei zu beachten ist, dass die Zellen zum längeren Überleben in Flüssigkeit oder in einem feuchten Medium eingebettet sein müssen.

Die lebenden Zellen 2 sind in einer Matrix eingebettet, die vorteilhafter Weise ein Nährmedium 3 enthält. Eine Vorrichtung zu Stabilisierung der Zellen und des Nährmediums 3 wird hier durch den Rahmen 6 dargestellt.

Ein notwendiger Zugang von Messgas 4 zu der sensitiven Schicht, dem Rezeptor, hier den lebenden Zellen 2, wird durch die Öffnungen oder die Durchgänge 11 gewährleistet. Diese können von unten her einen Zustrom von Messgas 4 zu den lebenden Zellen 2 durch den SI-Chip 1 ermöglichen. Zusätzlich können seitlich im Rahmen 6 Durchgänge 11 platziert sein. Wesentlich ist der minimierte Diffusionsweg durch eine die lebenden Zellen umgebende flüssige Substanz bzw. Eine entsprechend dünne Schicht dieser Substanz.

## Patentansprüche

1. Sensor zur Erfassung eines Gasgemisches, welches im Wesentlichen aus Luft besteht und ein oder mehrere Gase enthält, die auf Lebewesen eine Wirkung zeigen, bestehend aus:
- mindestens einem Sensorchip aus Silizium zur Auslesung von mindestens einem Signal, das an einer sensitiven Substanz bei vorliegendem einen oder mehreren Zielgasen im Messgas generiert wird,
- mindestens einer auf dem Sensorchip aufgebrachten sensitiven Substanz, bestehend aus lebenden Zellen, welche auf mindestens ein Zielgas anspricht/ansprechen,
- einer Signalverarbeitungseinheit zur Auswertung von Signalen.

2. Sensor nach Anspruch 1, der Vorrichtungen zur Versorgung der lebenden Zellen mit Nährstoffen und Feuchtigkeit aufweist, um die Lebensdauer der Zellen zu optimieren.

3. Sensor nach Anspruch 1 oder 2, der Vorrichtungen zur Versorgung der lebenden Zellen mit Licht aufweist, um die Lebensdauer der Zellen zu optimieren.

4. Sensor nach Anspruch 1, 2 oder 3, bei dem die lebenden Zellen auf dem Sensorchip in einer Gase durchlässigen Matrix eingebettet sind, die die Zellen auf der Sensoroberfläche fixiert.

5. Sensor nach Anspruch 2, 3 oder 4, der Vorrichtungen zur Versorgung der lebenden Zellen mit einem Wachstumshemmer, wie Zytostatika, aufweist, um eine Wucherung der Zellen zu verhindern.

6. Sensor nach Anspruch 2, 3 oder 4, der Vorrichtungen zur Versorgung der lebenden Zellen mit Bakteriziden, wie Antibiotika, und/oder Fungiziden, z.B. Antimykotika, aufweist, um die Ansiedelung von Fremdbakterien, Pilzen oder Sporen zu verhindern.

7. Sensor nach einem der vorhergehenden Ansprüche, der Vorrichtungen zur Versorgung der lebenden Zellen mit Natriumazid, Streptomycin, Penicillin enthält, um den Befall von Fremdbakterien zu verhindern.

8. Sensor nach einem der vorhergehenden Ansprüche, der Vorrichtungen zur Versorgung der lebenden Zellen mit Proteinen und Enzymen enthält, wie fetales Serum, normalen Serum oder Glutamin.

9. Sensor nach einem der vorhergehenden Ansprüche, der Vorrichtungen zum Einhalten einer konstanten vorgegebenen Temperatur des Zellchips enthält.

10. Sensor nach einem der vorhergehenden Ansprüche, der eine feinporige Membran mit einer Porenweite von etwa 0,1 µm - 10 µm aufweist, die den Zutritt von Messgas zum Sensor zulässt, jedoch zumindest Schadstoffe wie Staub, Aerosole oder Fremdbakterien und/oder Viren sperrt.

11. Sensor nach Anspruch 10, bei dem die feinporige Membran aus einem Polymer oder Polymergemischen aus polyfluoriertem Kohlenwasserstoff, Polypropylen, Polyethylen, Polyamid oder Polyimid besteht.

12. Sensor nach einem der vorhergehenden Ansprüche, bei dem sich die lebenden Zellen direkt auf bzw. in unmittelbarer Nähe des Chipbereiches befinden, so dass die Zellsignale auslesbar sind.

13. Sensor nach einem der vorhergehenden Ansprüche, bei dem Signale der lebenden Zellen über Elektroden, zwischen lebenden Zellen und Auslese-Chipbereich übertragbar sind.

14. Sensor nach einem der vorhergehenden Ansprüche, bei dem der Abstand zwischen lebenden Zellen und Messgas bzw. zwischen äußerem Rand einer die lebenden Zellen umgebenden Matrix und Messgas kleiner als 1mm ist.

15. Sensor nach Anspruch 14, bei dem der Abstand zwischen lebenden Zellen und Messgas bzw. zwischen äußerem Rand einer die lebenden Zellen umgebenden Flüssigkeit zum Messgas kleiner als 500 µm ist.

16. Sensor nach einem der vorhergehenden Ansprüche, bei dem auf der Rückseite des Sensorchips oder in Begrenzungen der sensitiven Substanz mindesten ein Durchgang (5,11) vorhanden ist.

17. Sensor nach Anspruch 16, bei dem der mindestens eine Durchgang einem Durchmesser von etwa 5-10 µm aufweist.

18. Sensor nach einem der vorhergehenden Ansprüche, bei dem mindestens ein enger lang gestreckter Fluidkanal mit einem Durchmesser im Bereich von 10 µm bis 1mm vorhanden ist, wodurch das in der Flüssigkeit gelöste Gas aktiv zu den Zellen transportiert wird.

19. Sensor nach Anspruch 18, bei dem sich eine von außen erzeugte Strömung der Flüssigkeit über den Sensorchip ausbildet.

20. Sensor nach einem der vorhergehenden Ansprüche, bei dem mehrere Sensorchips zu mindestens einer Gruppe zusammengefasst sind, wobei die Sensorchips jeweils andere lebende Zellen enthalten.

21. Sensor nach einem der Ansprüche 1-20 wobei mehrere Sensorchips vorhanden sind auf denen sich unterschiedliche Zelldichten befinden.

22. Sensor nach einem der Ansprüche 1-21 wobei sich unterschiedliche Zelllinien auf einem Chip befinden.

23. Verfahren zum Betrieb eines Sensors entsprechend einem der Ansprüche 1 - 22, bei dem ein Sensor mit einer Autokalibriereinheit ausgestattet um einen Nullpunkt in regelmäßigen Zeitabständen auf mindestens einem der Sensoren einzustellen.

24. Verfahren nach Anspruch 23, bei dem ein von Messgas unbelasteter Referenzsensor vorhanden ist, dessen Signale mit einem real messenden Sensor verglichen werden.

25. Verfahren zum Betrieb eines Sensors entsprechend einem der Ansprüche 18 oder 19, bei dem das Spülen der Zellen mit dem Fluid zwischenzeitlich unterbrochen wird.

26. Verfahren nach Anspruch 25, bei dem während der Unterbrechung des Spülens der Anstieg von Stoffwechselprodukten der Zellen gemessen wird.

27. Verfahren nach einem der Ansprüche 23 - 26, bei dem die mehreren Chips sequentiell betrieben werden und bei Erschöpfung der Messfähigkeit eines betriebenen Chips ein Wechsel auf einen anderen Chip geschieht.

28. Verfahren nach Anspruch 27, bei dem die Aktivierung des Betriebs eines Chips durch Wegnahme eines Wachstumshemmers geschieht.

29. Verfahren nach einem der Ansprüche 23 - 28, bei dem die Auswerteeinheit die Signale der unterschiedlichen Chips vergleicht und daraus weitergehende Informationen über die vorhandenen Gase/Gasgruppen gewinnt.
